# EUROPEAN PATENT SPECIFICATION

(11) **EP 1 545 276 B1**
(45) Date of publication and mention of the grant of the patent: **27.12.2006**
(21) Application number: 03750934.6
(22) Date of filing: 19.09.2003
(51) Int. Cl.: A47G 25/58, A61L 9/16, A47G 25/56

(54) **ODOUR ABSORBING CLOTHES COVER**
GERUCHSABSORBIERENDER KLEIDERSACK
HOUSSE POUR VETEMENTS ABSORBANT LES ODEURS

(30) Priority: 26.09.2002 GB 0222359
(43) Date of publication of application: 29.06.2005
(73) Proprietor: Reckitt Benckiser (UK) Limited, Slough, Berkshire SL1 3UH (GB)
(72) Inventor: Cornelius, Gay Joyce, Reckitt Benckiser Corp., Hull HU8 7DS, East Yorkshire (GB); Jones, Stuart Michael Ruan, Royston, Hertfordshire SG8 6UT (GB)
(74) Representative: Bowers, Craig Malcolm
(86) International application number: PCT/GB2003/004025
(87) International publication number: WO 2004/028317

(56) References cited:
- WO-A-02/055115
- US-A- 5 078 668
- US-A- 5 585 107
- PATENT ABSTRACTS OF JAPAN vol. 1998, no. 11, 30 September 1998 (1998-09-30) & JP 10 165288 A (EBARA CORP), 23 June 1998 (1998-06-23)
- PATENT ABSTRACTS OF JAPAN vol. 1996, no. 07, 31 July 1996 (1996-07-31) & JP 08 070981 A (DAIWABO CO LTD), 19 March 1996 (1996-03-19)
- PATENT ABSTRACTS OF JAPAN vol. 1997, no. 02, 28 February 1997 (1997-02-28) & JP 08 266388 A (ASAHI CHEM IND CO LTD), 15 October 1996 (1996-10-15)

## Description

This invention relates to odour absorbing clothes covers and methods of removing or mitigating odours from clothes.

Through everyday use, unpleasant or unwanted odours may become absorbed by clothing. Odours from sweat and smoke absorbed by clothing are a common problem. In many instances an item of clothing will be worn by a person, during which time odours are absorbed, and after which the item of clothing will be stored without washing it to remove the odours. When the item of clothing is worn for a second or subsequent occasion, very often the unpleasant or unwanted absorbed odours can be detected by the wearer or other persons close by. Generally items of clothing are stored in close proximity to one another, permitting odours to be transferred from an odour-emitting item of clothing to adjoining items of clothing (which may be clean items of clothing). Clothes which are often stored without washing between uses, and which tend to have absorbed odours, include suits, coats and knitwear.

There are products on the market such as spray and aerosol compositions which may be sprayed onto clothing to mask unpleasant or unwanted odours before storing the clothing. Such compositions only serve to hide the odours for a limited period of time and can, themselves, cause the clothing to take on an unwanted perfumed smell.

For footwear, products are known in the form of removable insoles, which include an odour absorbing substrate. When placed inside footwear, such insoles serve to absorb odours, as opposed to simply masking them.

There have been proposals, for example US 5 585 107, to provide odour absorbing bags for the receipt of clothing. The enclosed construction of such bags is not well suited for the receipt of larger items of clothing such as jackets and coats.

JP-A-10165288 discloses an odour absorbing, air-permeable clothes cover comprising an unwoven cloth sewed on three sides to form an envelope for an item of clothing. The lower envelope edge is open and the top edge comprises an opening for a hanger.

US-A-5078668 discloses a similar clothes cover comprising an elongate front slit covered by a flap.

It would be advantageous for a user to be able to remove unwanted or unpleasant odours from an item of clothing previously worn by the user, in a convenient manner, without the need to wash or dry clean the item of clothing before further use of the item.

It is therefore an aim of preferred embodiments of the present invention to overcome or mitigate problems of the prior art.

According to a first aspect of the invention there is provided an odour-absorbing clothes cover comprising an odour-absorbing sheet material, the sheet material defining an enclosure arranged, in use, to receive an item of clothing, the enclosure comprising front and rear surfaces connected or folded together along their side and upper peripheral edges but not along their lowermost peripheral edges such that, in use, an item of clothing placed into the enclosure defined between the front and rear surfaces may protrude through the lowermost peripheral opening thereby provided; wherein an opening is formed in the front surface of the enclosure, such opening extending directly from the lowermost peripheral opening, thus forming a pair of flaps in the front surface, which flaps may be drawn back by a user to aid insertion of an item of clothing into the enclosure.

Thus the flaps are not attached to the clothes cover at its lowermost periphery. Accordingly they may be drawn back to provide a wide opening through which large items of clothing, for example jackets and coats, may be passed.

The opening preferably extends to the uppermost peripheral edge, so that the flaps are entirely separate from each other. Alternatively the opening may terminate before reaching the uppermost peripheral edge, leaving a bridge or web of material between the flaps, extending between the uppermost peripheral edge and the upper end of the opening.

Suitably the sheet material is a non-woven sheet material, and is preferably a non-woven fibrous sheet material.

The sheet material is preferably a fibrous web or mat.

The sheet material is preferably an air permeable sheet material.

Suitable sheet materials include polyesters, polyamides, polyvinyl alcohols, cellulosics (for example rayon, viscose) and nylon, or mixtures thereof for example. The sheet material may alternatively comprise natural fibres, such as cotton, linen, flax and wool, or mixtures thereof, for example. The sheet material may comprise a mixture of synthetic and natural fibres.

Suitably the sheet material is comprised of synthetic fibres formed into a web, mat or similar flexible sheetform substrate. The sheet material may be a laminar composite material of layers of non-woven fibres, woven fibres or mixtures thereof which layers may comprise the same or different materials.

Preferred sheet materials comprise fibres of polyester or cellulose, including viscose and rayon.

Odour absorbing materials may be selected according to the intended use and odours to be absorbed, and include activated carbon, zeolites, inorganic compounds such as silica and metal oxides e.g. of titanium (TiO₂), zinc (ZnO) and aluminium and mixtures thereof, and may be in crystalline, microcrystalline, granular or other particulate form, for example.

Most preferred as an odour absorbing material is activated carbon, preferably in particulate form. The activated carbon may take the form of charcoal, peat, coconut shell, lignite or wood, for example but is preferably charcoal.

Particle sizes may depend on the material selected but will typically be between about 0.1µm to about 300µm, preferably up to about 75µm, and preferably not less than 1µm, more preferably not less than 25µm, these values being nominal (mean) diameters.

Suitably the odour-absorbing material is present in the sheet material in an amount of at least 5%(w/w), preferably at least 10%(w/w), more preferably at least 15%(w/w), still more preferably at least 20%(w/w), yet more preferably at least 25%(w/w), and most preferably at least 30%(w/w), of the total weight of the sheet material.

Suitably the odour-absorbing material is present in the sheet material in an amount of no more than 60%(w/w), preferably no more than 50%(w/w), more preferably no more than 45%(w/w), and most preferably no more than 40%(w/w), of the total weight of the sheet material.

Suitably the odour-absorbing material is retained or impregnated within the sheet material, preferably using a fixing agent, and whereby in use shedding of the odour-absorbing material is inhibited, preferably substantially avoided.

Suitably the fixing agent comprises a binder, and preferably a film-forming agent. Suitable binders include a latex, such as an acrylics or styrene butadiene latex or natural rubber based binder, especially containing a film former and/or an anti-foaming agent. The term film former means a material capable of forming a film when dry at ambient temperature and pressure. Suitable film-formers include polyvinyl alcohol or polyvinyl alcohol/vinyl acetate copolymers, and quaternary ammonium salts of polyvinylpyrrolidone/vinyl acetate copolymers.

Suitably impregnation of the sheet material with the odour-absorbing material is carried out using an odour-absorbing material-containing liquid preferably also comprising a fixing agent, by any one or more of the following methods:
saturation by soaking in a convenient manner e.g. simply delivery of the appropriate chemical treatment liquor from a hose over the sheet material;
impregnation by immersion cf the sheet material in a bath of the treatment liquor;
forced impregnation into the sheet material by application of the liquor under pressure;
pouring of the treatment liquor over the sheet material by a curtain-coating device situated over a progressively advancing web of sheet material to drench the sheet material;
spraying the treatment liquor upon the fibrous material;
or an equivalent treatment of a web or mat of the sheet material.

Suitably the fixing agent comprises at least 5%(w/w) of the total weight of the sheet material, preferably at least 10%(w/w), more preferably at least 15%(w/w) yet more preferably at least 20%(w/w) and most preferably at least 25%(w/w).

Suitably the fixing agent comprises up to 60%(w/w) of the total weight of the sheet material, preferably up to 50%(w/w), more preferably up to 45%(w/w), and most preferably up to 40%(w/w).

The sheet material and/or odour-absorbing agent may be as described in WO 98/30326.

Suitably the opening is sufficiently large to pass a large item of clothing, such as a jacket, without folding it.

Preferably the opening is at least 40 cm, more preferably at least 50 cm in length.

The opening in the front surface of the enclosure may comprise an elongate slit.

The opening may comprise a cover portion, arranged, in use, to conceal the opening but allow access to the opening.

When the opening is an elongate slit the cover portion may comprise a flap of sheet material connected at one side of the slit and extending across the slit. The cover member is preferably substantially equal in length to the slit.
The cover member may comprise releasable fastening means to fasten the cover member across the opening, until such time in use when it is desired to access the opening. The fastening means may comprise press studs, buttons, micro hooks/eyes (eg trade mark VELCRO) or adhesive (including low tack adhesive, and including single or double layer - ie adhesive-to-sheet material or adhesive-to-adhesive). Other fastening means may be employed. When the opening is an elongate slit, suitably one edge of the slit overlaps the other edge of the slit to form an integral cover member over the slit. The fastening means is then suitably beneath the cover member.

Alternatively the opening may include a slide fastener, for example an interlocking teeth (zip) fastener.

The front and rear surfaces may be integrally formed from the same sheet material, folded about a fold line, or fold lines, to form the surfaces, and connected together as required to form the enclosure. In a convenient arrangement having a slit which extends from the top to the bottom of the front surface, and the front surface is this divided into two panels or lapels, a single sheet may be cut and the panels and lapels formed by folding them against the rear surface.

Alternatively the front and rear surfaces are separate surfaces, connected together to form the enclosure.

Preferably the front and rear together define a slim enclosure in which the front and rear surfaces bear against an item of clothing within the enclosure.

Where the front and rear surfaces are connected together (as distinct from being co-formed) they may be heat sealed, stitched, stapled, glued, pinned or woven together, for example.

The front and rear surfaces may be connected or folded together along a portion of each of the side and upper peripheral edges, or along the entire length of such edges.

Preferably there is provided an uppermost peripheral opening dimensioned to allow the hook of a clothes hanger to be inserted through this peripheral opening in use. Whilst the peripheral opening is dimensioned to allow a large hook to be inserted through it, it is preferably smaller than head-sized. Thus, suitably the enclosure has no usable head hole.

Suitably the front and rear surfaces have a peripheral shape generally corresponding to the outline of an item of clothing to be covered by the clothes cover. Preferably the front and rear surfaces generally conform in shape to the peripheral shape of a jacket, coat, shirt or jumper. Preferably, however, the front and rear surfaces do not include sleeve shaped portions.

Preferably the cover is of similar size to, or preferably somewhat larger than, a jacket or jumper. Suitably it is at least 40 cm, preferably at least 50 cm, and most preferably at least 60 cm wide (at its maximum). Suitably it is at least 40 cm, preferably at least 60 cm, more preferably at least 70 cm, and most preferably at least 80 cm long (at its maximum).

Preferably the cover is not itself a garment or item of clothing.

According to a second aspect of the invention there is provided a method of removing an odour from an item of clothing, the method comprising:
(a) providing an odour-absorbing clothes cover of the first aspect of the invention; and
(b) inserting an item of clothing through the opening of the sheet material, into the enclosure.

When a peripheral opening suitable for a hook is present, the method may comprise placing the item of clothing on a clothes hanger before step (b), and inserting the hook of the clothes hanger through the uppermost peripheral opening, when present, to protrude outside of the cover.

Thus the cover, enclosing the item of clothing may then be hung on a suitable item, such as a rail in a wardrobe.

Before or after use the cover may be stored in an outer packaging cover. The outer packaging cover may be constructed from a resealable plastics material, for example, and may be constructed to prevent odours, moisture or dust in the air from being absorbed by the odour-absorbing material of the clothes cover, prior to its use, or between uses.

In order to better understand the various aspects of the invention and to show how embodiments of the same may be put into effect, the invention will now be described by way of example, with reference to the accompanying drawings in which:

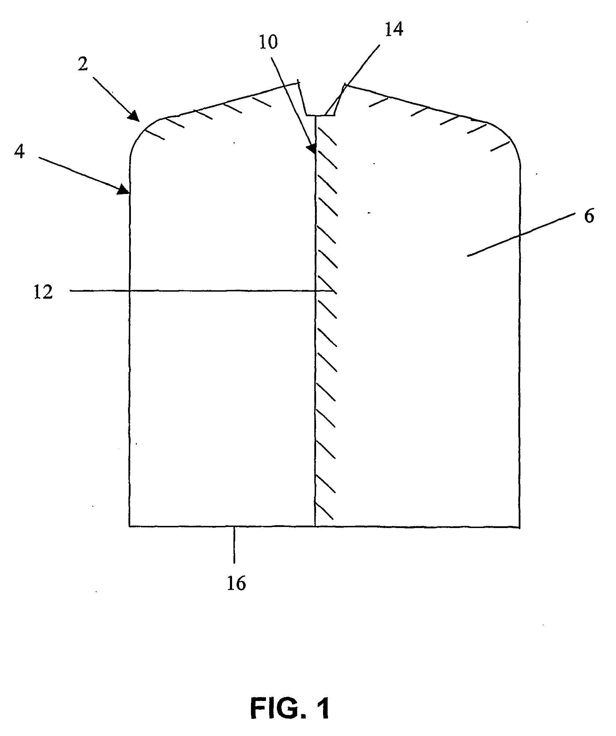

Figure 1 is a front view of a first embodiment of an odour-absorbing clothes cover of the invention;

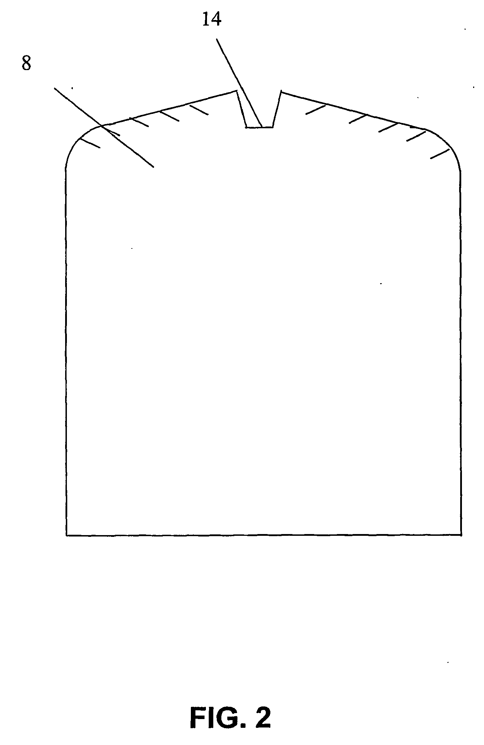

Figure 2 is a rear view of the embodiment shown in Figure 1;
Figure 3 is a view corresponding to that of Figure 1, with the opening thereinto partially exposed;
Figure 4 is a view of the first embodiment shown in Figure 1, into which a jacket has been partly inserted into the enclosure through an opening in the front surface of the cover; and

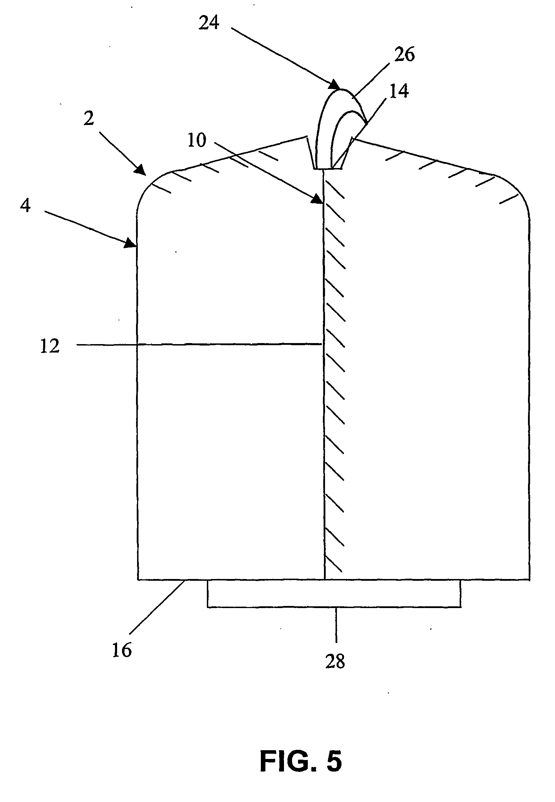

Figure 5 is a view corresponding to that of Figure 4, but with the jacket fully inserted within the enclosure.

An odour-absorbing clothes cover 2 comprises a sheet material 4 formed from connected front 6 and rear 8 surfaces. The front 6 and rear 8 surfaces are produced by folding a single sheet about two fold lines, which ultimately are the peripheral side edges of the cover. The peripheral edges are stitched together only at the shoulders. The result is a slim enclosure into which an item of clothing can be placed. An opening 10 is formed in the front surface 4, in the form of an elongate slit extending from the top of the front surface down to the lowermost peripheral edge thereof, and bisecting the front surface. The opening is overlapped by a covering flap 12. Beneath the covering flap 12 there is a single hook and loop (VELCRO trade mark) fastener (not shown) about half way up the covering flap.

The cover has an uppermost, short peripheral opening 14 cut from the junction between the front 6 and rear 8 surfaces at the upper edge of the sheet material 4, and a lowermost, long, peripheral opening 16. The lowermost edge 20 of the front surface is not connected to the lowermost edge 21 of the rear surface. Accordingly the lowermost opening 16 extends across the entire width of the cover.

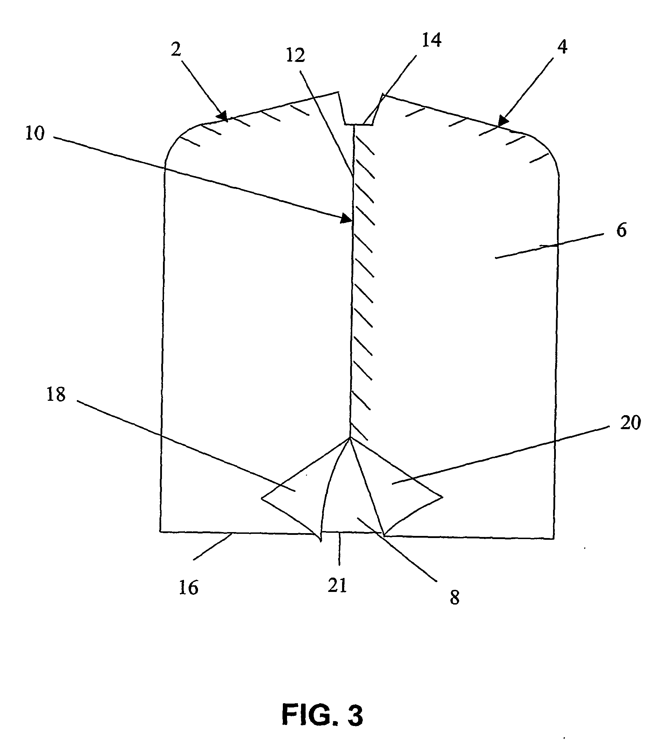

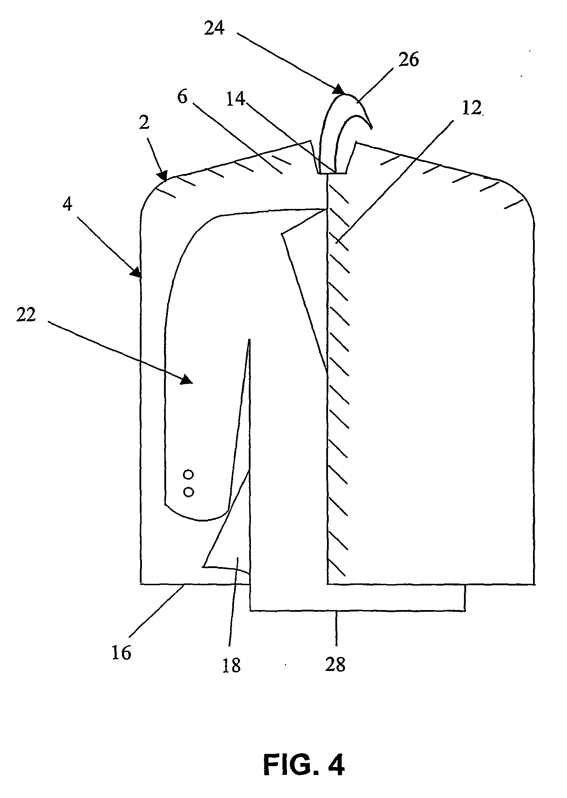

The opening 10 bisects the uppermost opening 14 and also bisects the lowermost opening 16. Thus, the opening 10 forms a curtain-like pair of flaps 18 and 20 in the front surface 6, which may be drawn aside (see Figures 3 and 4). This embodiment is useful for items of clothing which are relatively long, such as coats, dresses and jackets, which may be inserted into the opening 10 by pulling back the flaps 18 and 20 and pushing the item of clothing into the enclosure through the opening 10. The lowermost end of the item can protrude through the lowermost opening 16 thus preventing the item from becoming creased or crumpled whilst in the cover 2.

The clothes cover is 85 cm long and 65 cm wide (maximum length and width).

The uppermost peripheral opening 14 is sufficiently large to take the hook of a clothes hanger, but not so large that the cover does not cover the shoulders of an appropriate item of clothing - for example a jacket, jumper or shirt - inside the enclosure. Typically it is about 8-15 cm in width

The sheet material is constructed from polyester fibres having a length of 30-50mm formed into sheet material comprising a fibrous web, using the carding process of GB-A-2151667 and impregnated with a carbon-based odour-absorbing material, such as charcoal.

The sheet material is impregnated with activated charcoal by a process of immersing the sheet in a bath of treatment liquor in which activated charcoal is present. The treatment liquor, based on the dry weight under ambient temperature and pressure, comprises 25%(w/w) of an acrylic butadiene binder, 2%(w/w) of a polyvinyl alcohol film former, together forming a fixing agent, and an anti-foaming agent in a suitable organic carrier medium. The bulk of the liquor is made up of the particulate charcoal. During immersion of the sheet the charcoal is taken up into the fibrous web and held in place by the fixing agent to prevent future shedding. The sheet is then cut into appropriate shaped sheets in order to form enclosures. Activated charcoal constitutes 35%(w/w) of the finished sheet material. Fixing agent constitutes about 35%(w/w) of the finished sheet material.

In use, an item of clothing, in the form of a jacket 22, for example, is inserted into the enclosure formed by the connected front 6 and rear 8 surfaces by lifting the covering flap 12 of the opening 10 and inserting the jacket into the enclosure through the opening 10. If the jacket is mounted on a clothes hanger 24, as shown in Figure 4, the clothes hanger is also inserted through the opening 10 and the hook 26 of the clothes hanger pushed through the uppermost peripheral opening 14. Thus when the jacket 22 is fully inserted into the cover 2 the hook 26 protrudes from the uppermost peripheral opening 14 and the cover 2 and jacket 22 can be hung on a clothes rail, for example. It will be seen that in this embodiment the jacket hangs below the lowermost edge of the enclosure at 28, this edge being left unsewn to leave the opening 16.

In alternative embodiments the cover 2 may include a separate outer packaging cover (not shown) made from an impermeable plastics material, for example, and having a resealable opening. The packaging cover can protect the cover 2 and enclosed item from moisture, odours or dust in the atmosphere, at the point of sale and/or between uses. This can have the effect of prolonging the life-time of the odour-absorbing material in the cover 2 by preventing unnecessary odour-absorption or other absorption from the atmosphere, which may otherwise saturate the odour-absorbing material prematurely.

## Claims

1. An odour-absorbing clothes cover comprising an odour-absorbing sheet material, the sheet material defining an enclosure arranged, in use, to receive an item of clothing, the enclosure comprising front and rear surfaces connected or folded together along their side and upper peripheral edges but not along their lowermost peripheral edges such that, in use, an item of clothing placed into the enclosure defined between the front and rear surfaces may protrude through the lowermost peripheral opening thereby provided; wherein an opening is formed in the front surface of the enclosure, such opening extending directly from the lowermost peripheral opening, thus forming a pair of flaps in the front surface, which flaps may be drawn back by a user to aid insertion of an item of clothing into the enclosure.

2. A clothes cover as claimed in Claim 1, wherein the sheet material is a non-woven fibrous sheet material.

3. A clothes cover as claimed in Claim 1 or 2, wherein the sheet material is an air permeable sheet material.

4. A clothes cover as claimed in any preceding claim, wherein the sheet material is selected from a polyester, a polyamide, a polyvinyl alcohol, cellulose, nylon, and mixtures thereof.

5. A clothes cover as claimed in any preceding claim,
wherein the odour absorbing material is selected from activated carbon, a zeolite, titanium dioxide, zinc oxide, aluminium, and mixtures thereof.

6. A clothes cover as claimed in Claim 5, wherein the odour absorbing material is particulate activated carbon.

7. A clothes cover as claimed in any preceding claim, wherein the odour-absorbing material is present in the sheet material in an amount of at least 5%(w/w), preferably at least 30%(w/w), of the total weight of the sheet material.

8. A clothes cover as claimed in any preceding claim, wherein the odour-absorbing material is present in the sheet material in an amount of no more than 60%(w/w) of the total weight of the sheet material, preferably no more than 40%(w/w).

9. A clothes cover as claimed in any preceding claim,
wherein the odour-absorbing material comprises a fixing agent arranged to inhibit or substantially avoid shedding of the odour-absorbing material from the sheet material.

10. A clothes cover as claimed in any preceding claim,
wherein the opening in the surface of the enclosure comprises an elongate slit formed in the surface.

11. A clothes cover as claimed in Claim 10, wherein the slit extends in one direction at least in the region of the peripheral edge of the surface.

12. A clothes cover as claimed in any preceding claim,
wherein the opening comprises a cover member, arranged, in use, to close the opening intermittently.

13. A clothes cover as claimed in Claim 12, wherein the opening is an elongate slit, and the cover member comprises a flap of sheet material connected at one side of the slit and extending across the slit, being securable at that position but releasable therefrom.

14. A clothes cover as claimed in any preceding claim,
wherein the clothes cover comprises an uppermost peripheral opening, dimensioned to allow the hook of a clothes hanger to be inserted through it, in use.

15. A method of removing an odour from an item of clothing, the method comprising:
(a) providing an odour-absorbing clothes cover as claimed in any preceding claim; and
(b) inserting an item of clothing through the opening of the sheet material, into the enclosure.

## Patentansprüche

1. Geruchsabsorbierender Kleidersack, umfassend ein geruchsabsorbierendes Lagenmaterial, wobei das Lagenmaterial eine Hülle definiert, die bei Verwendung zum Aufnehmen eines Kleidungsgegenstands vorgesehen ist, wobei die Hülle eine Vorder- und eine Rückfläche umfasst, die entlang ihrer untersten peripheren Kanten derart miteinander verbunden oder gefaltet sind, dass bei Verwendung ein Kleidungsstück, der in die zwischen den Vorder- und Rückflächen definierte Hülle gegeben ist, durch die **dadurch** bereitgestellte unterste Öffnung herausragen kann; wobei eine Öffnung in der Vorderfläche der Hülle ausgebildet ist, wobei sich eine derartige Öffnung direkt von der untersten peripheren Öffnung erstreckt, wodurch ein Laschenpaar in der Vorderfläche gebildet wird, wobei die Laschen durch einen Anwender zurückgezogen werden können, um das Einlegen eines Kleidungsgegenstands in die Hülle zu unterstützen.

2. Kleidersack nach Anspruch 1, wobei das Lagenmaterial ein Vliesfaserlagenmaterial ist.

3. Kleidersack nach Anspruch 1 oder 2, wobei das Lagenmaterial ein luftdurchlässiges Lagenmaterial ist.

4. Kleidersack nach einem der vorangehenden Ansprüche, wobei das Lagenmaterial ausgewählt ist aus einem Polyester, einem Polyamid, einem Polyvinylalkohol, Cellulose, Nylon und Gemischen davon.

5. Kleidersack nach einem der vorangehenden Ansprüche, wobei das geruchsabsorbierende Material ausgewählt ist aus Aktivkohle, einem Zeolith, Titandioxid, Zinkoxid, Aluminium und Gemischen davon.

6. Kleidersack nach Anspruch 5, wobei das geruchsabsorbierende Material gekörnte Aktivkohle ist.

7. Kleidersack nach einem der vorangehenden Ansprüche, wobei das geruchsabsorbierende Material im Lagenmaterial in einer Menge von mindestens 5 Gew.-%, vorzugsweise mindestens 30 Gew.-% des Gesamtgewichts des Lagenmaterials vorliegt.

8. Kleidersack nach einem der vorangehenden Ansprüche, wobei das geruchsabsorbierende Material im Lagenmaterial in einer Menge von nicht mehr als 60 Gew.-%, vorzugsweise nicht mehr als 40 Gew.-% des Gesamtgewichts des Lagenmaterials vorliegt.

9. Kleidersack nach einem der vorangehenden Ansprüche, wobei das geruchsabsorbierende Material ein Fixiermittel umfasst, das zum Hemmen oder wesentlichen Vermeiden der Ablösung des geruchsabsorbierenden Materials von dem Lagenmaterial vorgesehen ist.

10. Kleidersack nach einem der vorangehenden Ansprüche, wobei die Öffnung in der Oberfläche der Hülle einen in der Oberfläche ausgebildeten länglichen Schlitz umfasst.

11. Kleidersack nach Anspruch 10, wobei sich der Schlitz zumindest im Bereich der peripheren Kante der Oberfläche in einer Richtung erstreckt.

12. Kleidersack nach einem der vorangehenden Ansprüche, wobei die Öffnung ein Abdeckelement umfasst, das bei Verwendung zum zeitweiligen Verschließen der Öffnung vorgesehen ist.

13. Kleidersack nach Anspruch 12, wobei die Öffnung ein länglicher Schlitz ist und das Abdeckelement eine Lasche aus einem Lagenmaterial umfasst, die mit einer Seite des Schlitzes verbunden ist und über den Schlitz herausragt, wobei sie an dieser Position befestigungsfähig, jedoch davon ablösbar ist.

14. Kleidersack nach einem der vorangehenden Ansprüche, wobei der Kleidersack eine oberste periphere Öffnung umfasst, die derart bemessen ist, dass bei Verwendung der Haken eines Kleiderbügels dort hindurch eingelegt werden kann.

15. Verfahren zum Entfernen von Geruch aus einem Kleidungsgegenstand, wobei das Verfahren umfasst:
(a) Bereitstellen eines geruchsabsorbierenden Kleidersacks nach einem der vorangehenden Ansprüche und
(b) Einlegen eines Kleidungsgegenstands durch die Öffnung des Lagenmaterials in die Hülle.

## Revendications

1. Housse pour vêtements absorbant les odeurs comprenant un matériau en feuilles absorbant les odeurs, le matériau en feuilles définissant un espace clos agencé, à l'utilisation, pour recevoir un vêtement, l'espace clos comprenant des surfaces avant et arrière reliées ou pliées l'une avec l'autre le long de leurs bords périphériques latéraux et supérieurs mais pas le long de leurs bords périphériques les plus bas de manière que, à l'utilisation, un vêtement placé dans l'espace clos défini entre les surfaces avant et arrière puisse saillir via l'ouverture périphérique ainsi ménagée le plus bas ; dans laquelle une ouverture est formée dans la surface avant de l'espace clos, une telle ouverture s'étendant directement depuis l'ouverture périphérique ménagée le plus bas, formant ainsi une paire de volets dans la surface avant, lesquels volets peuvent être repliés par un utilisateur pour faciliter l'insertion d'un vêtement dans l'espace clos.

2. Housse pour vêtements selon la revendication 1, dans laquelle le matériau en feuilles est un matériau en feuilles fibreux, non tissé.

3. Housse pour vêtements selon la revendication 1 ou 2, dans laquelle le matériau en feuilles est un matériau en feuilles perméable à l'air.

4. Housse pour vêtements selon l'une quelconque des revendications précédentes, dans laquelle le matériau en feuilles est sélectionné dans le groupe des polyester, polyamide, alcool polyvinylique, cellulose, nylon, et des mélanges de ceux-ci.

5. Housse pour vêtements selon l'une quelconque des revendications précédentes, dans laquelle le matériau absorbant les odeurs est sélectionné dans le groupe des charbon actif, zéolite, dioxyde de titane, oxyde de zinc, aluminium, et des mélanges de ceux-ci.

6. Housse pour vêtements selon la revendication 5, dans laquelle le matériau absorbant les odeurs est du charbon actif particulaire.

7. Housse pour vêtements selon l'une quelconque des revendications précédentes, dans laquelle le matériau absorbant les odeurs est présent dans le matériau en feuilles en une quantité d'au moins 5 % (masse pour masse), de préférence d'au moins 30 % (masse pour masse), du poids total du matériau en feuilles.

8. Housse pour vêtements selon l'une quelconque des revendications précédentes, dans laquelle le matériau absorbant les odeurs est présent dans le matériau en feuilles en une quantité n'excédant pas 60 % (masse pour masse) du poids total du matériau en feuilles, de préférence n'excédant pas 40 % (masse pour masse).

9. Housse pour vêtements selon l'une quelconque des revendications précédentes, dans laquelle le matériau absorbant les odeurs comprend un agent de fixation agencé pour empêcher ou substantiellement éviter la perte du matériau absorbant les odeurs depuis le matériau en feuilles.

10. Housse pour vêtements selon l'une quelconque des revendications précédentes, dans laquelle l'ouverture dans la surface de l'espace clos comprend une fente allongée formée dans la surface.

11. Housse pour vêtements selon la revendication 10, dans laquelle la fente s'étend dans une direction au moins dans la région du bord périphérique de la surface.

12. Housse pour vêtements selon l'une quelconque des revendications précédentes, dans laquelle l'ouverture comprend un élément couvrant agencé, à l'utilisation, pour fermer l'ouverture par intermittence.

13. Housse pour vêtements selon la revendication 12, dans laquelle l'ouverture est une fente allongée, et l'élément couvrant comprend un volet de matériau en feuilles relié à un côté de la fente et s'étendant en travers de la fente, pouvant être fixé en cette position mais étant détachable.

14. Housse pour vêtements selon l'une quelconque des revendications précédentes, dans laquelle la housse pour vêtements comprend une ouverture périphérique tout en haut, dimensionnée pour permettre au crochet d'un cintre d'être inséré via l'ouverture, à l'utilisation.

15. Procédé pour supprimer une odeur d'un vêtement, le procédé comprenant :
(a) la fourniture d'une housse pour vêtements absorbant les odeurs selon l'une quelconque des revendications précédentes ; et
(b) l'insertion d'un vêtement via l'ouverture du matériau en feuilles, dans l'espace clos.
